# EUROPEAN PATENT SPECIFICATION

(11) **EP 3 378 377 B1**
(45) Date of publication and mention of the grant of the patent: **07.07.2021**
(21) Application number: 17305330.7
(22) Date of filing: 23.03.2017
(51) Int. Cl.: A61B 3/00, A61B 3/11

(54) **METHOD FOR DETERMINING A RISK SCORE OF AN EYE DISEASE FOR A USER AND SYSTEM FOR THE EXECUTION OF SUCH METHOD**
VERFAHREN ZUR BESTIMMUNG EINER RISIKOEINSTUFUNG EINER AUGENKRANKHEIT FÜR EINEN BENUTZER UND SYSTEM ZUR AUSFÜHRUNG SOLCH EINES VERFAHRENS
PROCÉDÉ POUR DÉTERMINER UN SCORE DE RISQUE D'UNE MALADIE DE L' IL D'UN UTILISATEUR ET SYSTÈME D'EXÉCUTION DUDITCE PROCÉDÉ

(43) Date of publication of application: 26.09.2018
(73) Proprietor: Essilor International, 94220 Charenton-le-Pont (FR)
(72) Inventor: BARRAU, Coralie, 94220 CHARENTON-LE-PONT (FR); VILLETTE, Thierry, 94220 CHARENTON-LE-PONT (FR)
(74) Representative: Cabinet Novitech

(56) References cited:
- WO-A1-2010/044791
- WO-A1-2014/151114
- WO-A1-2015/017536
- US-A1- 2010 302 507

## Description

### FIELD OF THE INVENTION

The invention relates to a method, implemented by computer means, for determining a risk score of an eye disease for a user.

The invention further relates to a system for the execution of the method for determining a risk score of an eye disease for a user and to a program adapted to perform the method for determining a risk score of an eye disease for a user when installed and executed in the system.

### BACKGROUND OF THE INVENTION

Some eye diseases, for instance age-related macular degeneration ("AMD"), cause irreversible blindness of the persons having the eye disease.

For example, AMD is often diagnosed in an advanced stage. Most of the persons having an AMD are unaware that they have AMD until they are diagnosed with late-stage. Moreover, most of the persons having an AMD, when first diagnosed, already have an irreversible and significant loss of visual acuity. For diagnosed persons, behavior modification, nutritional supplementation, photo-protection, anti-vascular endothelial growth factor ("VEGF") treatment may be used to reduce the incidence of progression of irreversible visual loss.

Since some eye diseases progress quickly, there is a need for an early detection of these eye diseases. Early detection of eye diseases may offer the opportunity for early treatment and better possibilities for visual outcomes.

The most common approach in assessing retinal function for diagnosing AMD is visual acuity. However, the acuity testing is simple and quick, no information on retinal function in early AMD are provided, since the visual acuity changes due to early AMD are undetectable.

Hence, early eye disease, such as AMD, cannot be detected in daily life and during routine vision exams. The documents US 2010/302507 A1 and WO 2010/044791 A1 describe standard methods for determining a risk score of an eye disease for a user using a first and a second eye-mediated physiological parameter for evaluating a risk score.

Therefore, there is a need for a method for detecting early eye disease, and more precisely for assessing functional change in early eye disease to better monitor its progression.

One object of the invention is to provide such a method.

### SUMMARY OF THE INVENTION

To this end, the invention proposes a method implemented by computer means, for determining a risk score of an eye disease for a user, the method comprising:
- a first eye-mediated physiological parameter providing step, during which a first eye-mediated physiological parameter indicative of a first eye-mediated perception or behaviour of the user is provided,
- a second eye-mediated physiological parameter providing step, during which a second eye-mediated physiological parameter indicative of a second eye-mediated perception or behaviour of the user is provided, and
- a risk score determining step, during which the risk score of eye disease is determined based on the first eye-mediated physiological parameter and on the second eye-mediated physiological parameter.

Advantageously, the method of the invention allows calculating the early risk score of an eye disease based on multiple parameters. More precisely, the method of the invention allows detecting the first functional signs of early eye disease to establish a relevant and updatable risk score based on different parameters.

According to embodiments, the method for providing a set of data according to the invention may further comprise one or several of the following features according to any possible combination:
- the first eye-mediated physiological parameter relates to the dark adaptation of at least one eye of the user, and wherein the second eye-mediated physiological parameter relates to the pupil light reflex of at least one eye of the user and/or to the flicker sensitivity of at least one eye of the user; and/or
- the first eye-mediated physiological parameter provided during the first eye-mediated physiological parameter providing step and/or the second eye-mediated physiological parameter provided during the second eye-mediated physiological parameter providing step are determined by at least one test chosen among the list of tests consisting of:
   - determination of the transient pupil response;
   - determination of the latency to constriction;
   - determination of the constriction amplitude;
   - determination of the post illumination state;
   - determination of the pupil diameter after light offset;
   - determination of the phasic pupil response;
   - determination of the rod recovery on dark adaptation;
   - determination of the cone recovery on dark adaptation;
   - determination of the flicker detection threshold; and/or
- the method further comprises a third eye-mediated physiological parameter providing step, during which a third eye-mediated physiological parameter indicative of a third eye-mediated perception or behaviour of the user is provided, wherein the risk score of an eye disease is determined considering the third eye-mediated physiological parameter; and/or
- the third eye-mediated physiological parameter relates to the photosensitivity of at least one eye of the user and/or the color sensitivity of at least one eye of the user and/or the contrast sensitivity of at least one eye of the user and/or the glare recovery of at least one eye of the user and/or the shape deformation sensitivity of at least one eye of the user and/or the visual acuity of at least one eye of the user and/or the spatial contrast
sensitivity of at least one eye of the user and/or the macular pigment density of at least one eye of the user; and/or
- the third eye-mediated physiological parameter provided during the third eye-mediated physiological parameter providing step is determined by at least one test chosen among the list of tests consisting of:
   - determination of the visual acuity;
   - determination of the photosensitivity threshold;
   - determination of the cone contrast threshold;
   - determination of the cone recovery on glare recovery; and/or
- the method further comprises a life profile data providing step, during which life profile data relative to at least one parameter of the life profile of the user is provided, wherein the risk score of an eye disease is determined considering the life profile data; and/or
- the parameter of the life profile of the user relates to the age of the user and/or the gender of the user and/or the professional situation of the user and/or the personal situation of the user and/or the living place of the user and/or the general state of health of the user and/or the health history of the user and/or the health history of the family of the user and/or the food habits of the user and/or the physical activity habits of the user and/or the rhythm of life of the user; and/or
- the method further comprises an environment parameter providing step, during which an environment parameter indicative of the environment of the user is provided, wherein the risk score of an eye disease is determined considering the environment parameter; and/or
- the parameter of the environment of the user relates to the light exposure of the user and/or the radiance and/or the spectral emission and/or spatial distribution and/or history of exposure of the light received by the user; and/or
- the method further comprises an eye imaging parameter provided step, during which an eye imaging parameter indicative of an eye imaging of the at least one eye of the user is provided, wherein the risk score of an eye disease is determined considering the eye imaging parameter; and/or
- the eye imaging parameter relates to at least a feature of a drusen of an eye of the wearer, said feature comprising at least one of the size and shape of the drusen, the total area of the druses and the location of the drusen; and/or
- the method further comprises an updating step, during which the first eye-mediated physiological parameter and/or the second eye-mediated physiological parameter and/or the third eye-mediated physiological parameter and/or the life profile data and/or the environment data and/or the eye imaging parameter are updated, wherein the risk score of an eye disease is determined considering the updated data.

Advantageously, the method of the invention allows updating the early risk score of an eye disease based on multiple parameters. In other words, the method of the invention allows providing to the user an accurate and updated value of the risk score.

Advantageously, the method of the invention allows helping the user to be aware of the effect of different parameters on his risk score and having the user either changing his habits or on the contrary amplifying some behaviour or habits.

The invention further relates to a system for the execution of the method for determining a risk score of an eye disease for a user according to the invention.

The invention further relates to a program adapted to perform the method for determining a risk score of an eye disease for a user according to the invention when installed and executed in the system of the invention.

The invention further relates to a system for determining a risk score of an eye disease for a user, the system comprising:
- a first eye-mediated physiological parameter providing means for providing a first eye-mediated physiological parameter indicative of a first eye-mediated perception or behaviour of the user,
- a second eye-mediated physiological parameter providing means for providing a second eye-mediated physiological parameter indicative of a second eye-mediated perception or behaviour of the user, and
- a risk score determining means for determining the risk score of eye disease based on the first eye-mediated physiological parameter and on the second eye-mediated physiological parameter.

The invention further relates to a computer program product comprising one or more stored sequences of instructions that are accessible to a processor and which, when executed by the processor, causes the processor to carry out the steps of the method according to the invention.

The invention further relates to a system for determining a risk score of an eye disease for a user comprising a processor adapted to store one or more sequences of instruction and to carry out at least one of the steps of the method according to the invention.

The invention further relates to a computer readable storage medium having a program recorded thereon, where the program makes the computer execute the steps of the method according to the invention.

### BRIEF DESCRIPTION OF THE DRAWINGS

Other characteristics and advantages of the invention will become more apparent from the claims and from the following description of some embodiments given by way of example without limitation with reference to the drawing:

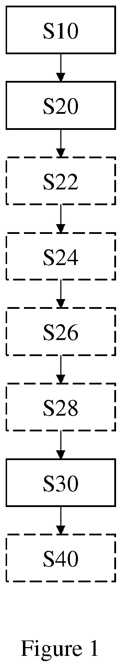

- Figure 1 is a flowchart of the different steps of a method for determining a risk score of an eye disease for a user according to the invention.

### DETAILED DESCRIPTION OF PREFERRED EMBODIMENTS

The invention relates to a method, for example implemented by computer means, for determining a risk score of an eye disease for a user.

For instance, the method allows determining a risk score of age-related macular degeneration.

A flowchart of the different steps of the method for determining a risk score of an eye disease for a user according to the invention is represented in figure 1.

The method comprises a first eye-mediated physiological parameter providing step (S10) and a second eye-mediated physiological parameter providing step (S20).

During the first eye-mediated physiological parameter providing step (S10), a first eye-mediated physiological parameter indicative of a first eye-mediated perception or behaviour of the user is provided.

The first eye-mediated physiological parameter may relate to the dark adaptation of at least one eye of the user.

During the second eye-mediated physiological parameter providing step (S20), a second eye-mediated physiological parameter indicative of a second eye-mediated perception or behaviour of the user is provided.

The second eye-mediated physiological parameter may relate to the pupil light reflex of at least one eye of the user and/or to the flicker sensitivity of at least one eye of the user.

The first eye-mediated physiological parameter provided during the first eye-mediated physiological parameter providing step (S10) may be determined by at least one test. Likewise, the second eye-mediated physiological parameter provided during the second eye-mediated physiological parameter providing step (S20) may be determined by at least one test.

The tests for determining the first and/or second eye-mediated physiological parameters may be chosen among the list of tests consisting of: the determination of the transient pupil response, the determination of the latency to constriction, the determination of the constriction amplitude, the determination of the post illumination state, the determination of the pupil diameter after light offset, the determination of the phasic pupil response, the determination of the rod recovery on dark adaptation, the determination of the cone recovery on dark adaptation, the determination of the flicker detection threshold.

The dark adaptation may be determined using measurements of the rod recovery on dark adaptation or of the cone recovery on dark adaptation. This test is reproducible and has a good diagnostic capacity.

The tests for determining the dark adaptation of at least one eye of the user may be performed with low light levels.

The pupil light reflex may be determined using pupil recordings with sinusoidal light excitations, for instance at around 480 nm, namely at high excitation of the intrinsically photosensitive retinal ganglion cells ("ipRGC") and at around 630 nm, or with sinusoidal light stimulus, for instance 11.9 seconds sinusoidal stimulus. The pupil light reflex may be determined a priori to the illumination and after the illumination. The pupil light reflex may be determined during different duration of stimulation. The pupil light reflex may be determined using multifocal pupillography or irradiances bellow and above the melanopsin threshold to detect both deficits in rods and cones functions and deficits in ipRGC function. This test is quick, objective, non-invasive and reproducible.

The flicker sensitivity may be determined using a flickering stimulus. This test is quick, for instance around 4 to 7 minutes, reproducible, clinically applicable and has a good diagnostic capacity.

The first and second eye-mediated physiological parameters may be static parameters or dynamic parameters. Moreover, one among the first and second eye-mediated physiological parameters may be a static parameter, and the other among the first and second eye-mediated physiological parameters may be dynamic parameter.

The method comprises a risk score determining step (S30), during which the risk score of eye disease is determined based on the first eye-mediated physiological parameter and on the second eye-mediated physiological parameter.

Advantageously, the determination of the risk score of eye disease based on a plurality of parameters allows improving the early detection of eye disease.

The risk score of eye disease may be determined based on a combination of static and dynamic parameters.

The method may comprise a third eye-mediated physiological parameter providing step (S22), during which a third eye-mediated physiological parameter indicative of a third eye-mediated perception or behaviour of the user is provided.

The third eye-mediated physiological parameter may relate to the photosensitivity of at least one eye of the user and/or the color sensitivity of at least one eye of the user and/or the contrast sensitivity of at least one eye of the user and/or the shape deformation sensitivity of at least one eye of the user and/or the visual acuity of at least one eye of the user and/or the spatial contrast sensitivity of at least one eye of the user and/or the macular pigment density of at least one eye of the user.

The third eye-mediated physiological parameter may relate to the glare recovery of at least one eye of the user and/or to the photo-stress recovery of at least one eye of the user.

The third eye-mediated physiological parameter may relate to the chromatic function of at least one eye of the user and/or the visual distortion of at least one eye of the user and/or the low contrast visual acuity of at least one eye of the user.

The third eye-mediated physiological parameter provided during the third eye-mediated physiological parameter providing step (S22) may be determined by at least one test.

The tests for determining the third eye-mediated physiological parameter may be chosen among the list of tests consisting of: the determination of the visual acuity, the determination of the photosensitivity threshold, the determination of the cone contrast threshold, the determination of the cone recovery on glare recovery.

The list of tests for determining the third eye-mediated physiological parameter may comprise the determination of the colour sensitivity threshold. This test is fast and user-friendly.

The list of tests for determining the third eye-mediated physiological parameter may comprise the determination of the photo-stress recovery and/or the determination of the glare recovery. These tests are fast and user-friendly.

The photosensitivity threshold may be determined during dynamic automatized light sequences, for instance with 10 seconds per steps, 50 lux step from 0 to 1000 lux, or 100 lux step from 1000 to 2000 lux, or 200 lux step from 2000 to 4000 lux, or 400 lux step from 4000 to 8000 lux. The photosensitivity threshold may be determined during random fixed light levels sequences. The photosensitivity threshold may be determined using a portative and uniform lighting sphere, or using ultra-violet ("UV") light-emitting diodes ("LED") for progressive activation of photochromic lenses. The photosensitivity threshold may be determined using correlated colour-temperature ("CCT") from a natural white light, for instance 4000 K, to a very cold white light, for instance greater than 6500 K. This test is easy to implement and clinically applicable.

The contrast sensitivity may be determined using a cone contrast threshold.

The visual distortion may be determined using Amsler grid test.

The third eye-mediated physiological parameter may be a static parameter or a dynamic parameter.

The risk score of an eye disease may be determined considering the third eye-mediated physiological parameter.

The method may comprise a life profile data providing step (S24), during which life profile data relative to at least one parameter of the life profile of the user is provided.

The parameter of the life profile of the user may relate to the age of the user and/or the gender of the user and/or the professional situation of the user and/or the personal situation of the user and/or the living place of the user and/or the general state of health of the user and/or the physical activity habits of the user.

The parameter of the life profile of the user may relate to the health history of the user, for instance to photosensitive treatments or to cataract surgery of at least one eye of the user.

The parameter of the life profile of the user may relate to the ethnicity of the user.

The parameter of the life profile of the user may relate to the health history of the family of the user, for example if parents of the user have an eye disease, such as AMD. The parameter of the life profile of the user may relate to the genetics of the user.

The parameter of the life profile of the user may relate to the food habits of the user and/or the rhythm of life of the user, for instance if the user smokes or the diet of the user.

The parameter of the life profile of the user may relate to oxidative stressors, such as a smoking treatment, a diet poor in antioxidants, a light treatment or a photosensitive treatment.

The parameter of the life profile of the user may relate to the protections used by the user, namely if the user uses sunglasses or hats.

The parameter of the life profile of the user may be determined with a questionnaire.

The risk score of an eye disease may be determined considering the life profile data.

The method may comprise an environment parameter providing step (S26), during which an environment parameter indicative of the environment of the user is provided.

The parameter of the environment of the user may relate to the light exposure of the user and/or the radiance and/or the spectral emission and/or spatial distribution and/or history of exposure of the light received by the user.

The light exposure of the user may be defined by the correlation of the number of light sources and/or the localization of the light sources and/or the spatial distribution of the light sources and/or the radiance of the light sources including the directivity of the light and/or the spectral distribution of the light sources and/or the exposure duration of the user and/or the repetition of the exposure of the user.

The light exposure of the user may be determined with a connected eyewear comprising spectrometers and/or colored photodiodes, such as UV, blue, green, red, and infra-red ("IR") photodiodes.

The risk score of an eye disease may be determined considering the environment parameter.

The method may comprise an eye imaging parameter provided step (S28), during which an eye imaging parameter indicative of an eye imaging of the at least one eye of the user is provided.

The eye imaging parameter may relate to at least a feature of a drusen of an eye of the wearer. The feature of a drusen of an eye of the wearer may comprise at least one of the size and shape of the drusen, the total area of the druses and the location of the drusen.

The feature of a drusen of an eye of the wearer may comprise the type of the drusen, for instance a hard type drusen, when the size of the drusen is smaller than 63 µm, an intermediate soft type drusen, when the size of the drusen is comprised between 63 µm and 125 µm, a large semi-solid type drusen, when the size of the drusen is greater than 125 µm.

The eye imaging parameter may relate to pigmentary abnormalities of at least one eye of the user and/or a geographic atrophy of at least one eye of the user and/or a choroidal neovascularization of at least one eye of the user.

The risk score of an eye disease may be determined considering the eye imaging parameter.

In particular, an age-related eye disease study risk ("AREDS") may be only based on an eye imaging parameter. For instance, the AREDS may comprise scores between 1 and 4. The score 1 corresponds to no drusen in at least one eye of the user or the size of the drusen is smaller than 63 µm and the total area of the druses is smaller than 125 µm. The score 2 corresponds to no geographic atrophy and the size of the drusen is greater than or equal to 63 µm and smaller than 125 µm and the total area of the druses is greater than or equal to 125 µm. The score 3a corresponds to soft distinct drusen, the size of the drusen is greater than or equal to 63 µm and smaller than 125 µm and the total area of the druses is greater than 360 µm. The score 3b corresponds to soft indistinct drusen, the size of the drusen is greater than or equal to 63 µm and smaller than 125 µm and the total area of the druses is greater than or equal to 656 µm. The score 4 corresponds to geographic atrophy or choroidal neovascularization and a visual acuity smaller than 20/32.

The method may further comprise an updating step (S40).

During the updating step (S40), the first eye-mediated physiological parameter and/or the second eye-mediated physiological parameter and/or the third eye-mediated physiological parameter and/or the life profile data and/or the environment data and/or the eye imaging parameter are updated.

The risk score of an eye disease may be determined considering the updated data.

Advantageously, the updating of the risk score of an eye disease of a user allows providing to the user an accurate and updated value of the risk score. More precisely, this updating allows helping the user to be aware of the effect of different parameters on his risk score and having the user either changing his habits or on the contrary amplifying some behaviour or habits.

The first eye-mediated physiological parameter and/or the second eye-mediated physiological parameter and/or the third eye-mediated physiological parameter and/or the life profile data and/or the environment data and/or the eye imaging parameter may be personalized according to the user and/or to the activities of the user and/or to the goals and performances to be reached by the user and/or to the user's doctor and/or to the user's localization.

The invention further relates to a system for the execution of the method for determining a risk score of an eye disease for a user as described previously.

The system may comprise a sensor or a plurality of sensors for measuring the first eye-mediated physiological parameter and/or the second eye-mediated physiological parameter and/or the third eye-mediated physiological parameter and/or the environment data and/or the eye imaging parameter.

After determining the risk score of an eye disease for a user, the system may activate specific functions depending on the risk score determined. For instance, a specific function to be activated may be an automation of home automation or of a vehicle, or the trigger of an alarm at the medical centre of the user.

The invention has been described above with the aid of embodiments without limitation of the general inventive concept. Moreover, the embodiments of the invention may be combined without any restriction.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A method, implemented by computer means, for determining a risk score of an eye disease for a user, the method comprising:
- **a first eye-mediated physiological parameter providing step** (S10), during which a first eye-mediated physiological parameter indicative of a first eye-mediated perception or behaviour of the user is provided,
- **a second eye-mediated physiological parameter providing step** (S20), during which a second eye-mediated physiological parameter indicative of a second eye-mediated perception or behaviour of the user is provided,
- **a life profile data providing step** (S24), during which life profile data relative to at least one parameter of the life profile of the user is provided, and
- **a risk score determining step** (S30), during which the risk score of eye disease is determined based on the first eye-mediated physiological parameter, the second eye-mediated physiological parameter and the life profile data.

2. The method according to claim 1, wherein the first eye-mediated physiological parameter relates to the dark adaptation of at least one eye of the user, and wherein the second eye-mediated physiological parameter relates to the pupil light reflex of at least one eye of the user and/or to the flicker sensitivity of at least one eye of the user.

3. The method according to claims 1 or 2, wherein the first eye-mediated physiological parameter provided during the first eye-mediated physiological parameter providing step and/or the second eye-mediated physiological parameter provided during the second eye-mediated physiological parameter providing step are determined by at least one test chosen among the list of tests consisting of:
- determination of the transient pupil response;
- determination of the latency to constriction;
- determination of the constriction amplitude;
- determination of the post illumination state;
- determination of the pupil diameter after light offset;
- determination of the phasic pupil response;
- determination of the rod recovery on dark adaptation;
- determination of the cone recovery on dark adaptation;
- determination of the flicker detection threshold.

4. The method according to any of the preceding claims, further comprising:
- **a third eye-mediated physiological parameter providing step** (S22), during which a third eye-mediated physiological parameter indicative of a third eye-mediated perception or behaviour of the user is provided,
wherein the risk score of an eye disease is determined considering the third eye-mediated physiological parameter.

5. The method according to claim 4, wherein the third eye-mediated physiological parameter relates to the photosensitivity of at least one eye of the user and/or the color sensitivity of at least one eye of the user and/or the contrast sensitivity of at least one eye of the user and/or the glare recovery of at least one eye of the user and/or the shape deformation sensitivity of at least one eye of the user and/or the visual acuity of at least one eye of the user and/or the spatial contrast sensitivity of at least one eye of the user and/or the macular pigment density of at least one eye of the user.

6. The method according to claims 4 or 5, wherein the third eye-mediated physiological parameter provided during the third eye-mediated physiological parameter providing step is determined by at least one test chosen among the list of tests consisting of:
- determination of the visual acuity;
- determination of the photosensitivity threshold;
- determination of the cone contrast threshold;
- determination of the cone recovery on glare recovery.

7. The method according to claim 1, wherein the parameter of the life profile of the user relates to the age of the user and/or the gender of the user and/or the professional situation of the user and/or the personal situation of the user and/or the living place of the user and/or the general state of health of the user and/or the health history of the user and/or the health history of the family of the user and/or the food habits of the user and/or the physical activity habits of the user and/or the rhythm of life of the user.

8. The method according to any of the preceding claims, further comprising:
- **an environment parameter providing step** (S26), during which an
environment parameter indicative of the environment of the user is provided, wherein the risk score of an eye disease is determined considering the environment parameter.

9. The method according to claim 8, wherein the parameter of the environment of the user relates to the light exposure of the user and/or the radiance and/or the spectral emission and/or spatial distribution and/or history of exposure of the light received by the user.

10. The method according to any of the preceding claims, further comprising:
- **an eye imaging parameter provided step** (S28), during which an eye imaging parameter indicative of an eye imaging of the at least one eye of the user is provided,
wherein the risk score of an eye disease is determined considering the eye imaging parameter.

11. The method according to claim 10, wherein the eye imaging parameter relates to at least a feature of a drusen of an eye of the wearer, said feature comprising at least one of the size and shape of the drusen, the total area of the druses and the location of the drusen.

12. The method according to any of the preceding claims, further comprising:
- **an updating step** (S40), during which the first eye-mediated physiological parameter and/or the second eye-mediated physiological parameter and/or the third eye-mediated physiological parameter and/or the life profile data and/or the environment data and/or the eye imaging parameter are updated,
wherein the risk score of an eye disease is determined considering the updated data.

13. A system for the execution of the method for determining a risk score of an eye disease for a user according to any of claims 1 to 12.

14. A program adapted to perform the method for determining a risk score of an eye disease for a user according to any of claims 1 to 12 when installed and executed in the system of claim 13.

## Patentansprüche

1. Durch Computermittel implementiertes Verfahren zur Bestimmung einer Risikoeinstufung einer Augenerkrankung für einen Benutzer, wobei das Verfahren umfasst:
- **einen ersten augenvermittelten physiologischen Parameter bereitstellenden Schritt** (S10), währenddessen ein erster augenvermittelter physiologischer Parameter bereitgestellt wird, der eine erste augenvermittelte Wahrnehmung oder ein erstes augenvermitteltes Verhalten des Benutzers angibt,
- **einen zweiten augenvermittelten physiologischen Parameter bereitstellenden Schritt** (S20), währenddessen ein zweiten augenvermittelter physiologischer Parameter bereitgestellt wird, der eine zweite augenvermittelte Wahrnehmung oder ein zweites augenvermitteltes Verhalten des Benutzers angibt,
- **einen Lebensprofildaten bereitstellenden Schritt** (S24), währenddessen Lebensprofildaten, die sich auf mindestens einen Parameter des Lebensprofils des Benutzers beziehen, bereitgestellt werden, und
- **einen Risikoeinstufung bestimmenden Schritt** (S30), währenddessen die Risikoeinstufung der Augenerkrankung basierend auf dem ersten augenvermittelten physiologischen Parameter, dem zweiten augenvermittelten physiologischen Parameter und den Lebensprofildaten bestimmt wird.

2. Verfahren nach Anspruch 1, wobei der erste augenvermittelte physiologische Parameter die Dunkeladaptation von mindestens einem Auge des Benutzers betrifft, und wobei der zweite augenvermittelte physiologische Parameter den Pupillenlichtreflex von mindestens einem Auge des Benutzers und/oder die zeitliche Kontrastsensitivität (Flicker Sensitivity) von mindestens einem Auge des Benutzers betrifft.

3. Verfahren nach den Ansprüchen 1 oder 2, wobei der erste augenvermittelte physiologische Parameter, der während des ersten augenvermittelten physiologischen Parameter bereitstellenden Schritts bereitgestellt wird, und/oder der zweite augenvermittelte physiologische Parameter, der während des zweiten augenvermittelten physiologischen Parameter bereitstellenden Schritts bereitgestellt wird, durch mindestens einen Test ausgewählt aus der Liste von Tests bestimmt wird bzw. werden, die aus folgenden besteht:
- Bestimmung der transienten Pupillenreaktion;
- Bestimmung der Latenzzeit bis zur Konstriktion;
- Bestimmung der Konstriktionsamplitude;
- Bestimmung des Zustands nach Beleuchten;
- Bestimmung des Pupillendurchmessers nach Lichtversatz;
- Bestimmung der phasischen Pupillenreaktion;
- Bestimmung der Stäbchenerholung bei Dunkeladaptation;
- Bestimmung der Zapfenerholung bei Dunkeladaptation;
- Bestimmung des Flickerdetektierungsschwellenwerts.

4. Verfahren nach einem der vorhergehenden Ansprüche, das des Weiteren umfasst:
- **einen dritten augenvermittelten physiologischen Parameter bereitstellenden Schritt** (S22), währenddessen ein dritter augenvermittelter physiologischer Parameter bereitgestellt wird, der eine dritte augenvermittelte Wahrnehmung oder ein drittes augenvermitteltes Verhalten des Benutzers angibt,
wobei die Risikoeinstufung einer Augenerkrankung unter Berücksichtigung des dritten augenvermittelten physiologischen Parameters bestimmt wird.

5. Verfahren nach Anspruch 4, wobei der dritte augenvermittelte physiologische Parameter die Lichtempfindlichkeit von mindestens einem Auge des Benutzers und/oder die Farbempfindlichkeit von mindestens einem Auge des Benutzers und/oder die Kontrastempfindlichkeit von mindestens einem Auge des Benutzers und/oder die Blenderholung von mindestens einem Auge des Benutzers und/oder die Formabweichungsempfindlichkeit von mindestens einem Auge des Benutzers und/oder die Sehschärfe von mindestens einem Auge des Benutzers und/oder die räumliche Kontrastempfindlichkeit von mindestens einem Auge des Benutzers und/oder die Makulapigmentdichte von mindestens einem Auge des Benutzers betrifft.

6. Verfahren nach den Ansprüchen 4 oder 5, wobei der dritte augenvermittelte physiologische Parameter, der während des dritten augenvermittelten physiologischen Parameter bereitstellenden Schritts bereitgestellt wird, durch mindestens einen Test ausgewählt aus der Liste von Tests bestimmt wird, die aus folgenden besteht:
- Bestimmung der Sehschärfe;
- Bestimmung des Lichtempfindlichkeitsschwellenwerts;
- Bestimmung des Zapfenkontrastschwellenwerts;
- Bestimmung der Zapfenerholung bei Blenderholung.

7. Verfahren nach Anspruch 1, wobei der Parameter des Lebensprofils des Benutzers sich auf das Alter des Benutzers und/oder das Geschlecht des Benutzers und/oder die berufliche Situation des Benutzers und/oder die persönliche Situation des Benutzers und/oder den Lebensort des Benutzers und/oder den allgemeinen Gesundheitszustand des Benutzers und/oder die gesundheitliche Vorgeschichte des Benutzers und/oder die gesundheitliche Vorgeschichte der Familie des Benutzers und/oder die Ernährungsgewohnheiten des Benutzers und/oder die physischen Aktivitätsgewohnheiten des Benutzers und/oder den Lebensrhythmus des Benutzers bezieht.

8. Verfahren nach einem der vorhergehenden Ansprüche, das des Weiteren umfasst:
- **einen Umgebungsparameter bereitstellenden Schritt** (S26), währenddessen ein Umgebungsparameter bereitgestellt wird, der die Umgebung des Benutzers angibt,
wobei die Risikoeinstufung einer Augenerkrankung unter Berücksichtigung des Umgebungsparameters bestimmt wird.

9. Verfahren nach Anspruch 8, wobei der Parameter der Umgebung des Benutzers sich auf die Lichtexposition des Benutzers und/oder die Strahldichte und/oder die Spektralemission und/oder räumliche Verteilung und/oder Vorgeschichte der Exposition des Lichts, welche der Benutzer empfangen hat, bezieht.

10. Verfahren nach einem der vorhergehenden Ansprüche, das des Weiteren umfasst:
- **einen Augenbildgebungsparameter bereitstellenden Schritt** (S28), währenddessen ein Augenbildgebungsparameter bereitgestellt wird, der eine Augenbildgebung von mindestens einem Auge des Benutzers angibt,
wobei die Risikoeinstufung einer Augenerkrankung unter Berücksichtigung des Augenbildgebungsparameters bestimmt wird.

11. Verfahren nach Anspruch 10, wobei der Augenbildgebungsparameter mindestens ein Merkmal einer Druse eines Auges des Trägers betrifft, wobei das Merkmal mindestens eines von der Größe und Form der Druse, der Gesamtfläche der Drusen und der Position der Druse betrifft.

12. Verfahren nach einem der vorhergehenden Ansprüche, das des Weiteren umfasst:
- **einen Aktualisierungsschritt** (S40), währenddessen der erste augenvermittelte physiologische Parameter und/oder der zweite augenvermittelte physiologische Parameter und/oder der dritte augenvermittelte physiologische Parameter und/oder die Lebensprofildaten und/oder die Umgebungsdaten und/oder die Augenbildgebungsparameter aktualisiert werden,
wobei die Risikoeinstufung einer Augenerkrankung unter Berücksichtigung der aktualisierten Daten bestimmt wird.

13. System zur Ausführung des Verfahrens zur Bestimmung einer Risikoeinstufung einer Augenerkrankung für einen Benutzer gemäß einem der Ansprüche 1 bis 12.

14. Programm, das zur Ausführung des Verfahrens zur Bestimmung einer Risikoeinstufung einer Augenerkrankung für einen Benutzer gemäß einem der Ansprüche 1 bis 12 eingerichtet ist, wenn es in dem System gemäß Anspruch 13 installiert und ausgeführt wird.

## Revendications

1. Procédé, mis en œuvre par des moyens informatiques, pour déterminer un score de risque d'une maladie de l'œil pour un utilisateur, le procédé comprenant :
- une étape de fourniture d'un premier paramètre physiologique médié par l'œil (S10), au cours de laquelle un premier paramètre physiologique médié par l'œil indiquant une première perception ou un premier comportement médié par l'œil de l'utilisateur est fourni,
- une étape de fourniture d'un deuxième paramètre physiologique médié par l'œil (S20), au cours de laquelle un deuxième paramètre physiologique médié par l'œil indiquant une deuxième perception ou un deuxième comportement médié par l'œil de l'utilisateur est fourni,
- une étape de fourniture de données de profil de vie (S24), au cours de laquelle des données de profil de vie relatives à au moins un paramètre du profil de vie de l'utilisateur sont fournies, et
- une étape de détermination du score de risque (S30), au cours de laquelle le score de risque de maladie de l'œil est déterminé sur la base du premier paramètre physiologique médié par l'œil, du deuxième paramètre physiologique médié par l'œil et des données de profil de vie.

2. Procédé selon la revendication 1, le premier paramètre physiologique médié par l'œil concernant l'adaptation à l'obscurité d'au moins un œil de l'utilisateur, et le deuxième paramètre physiologique médié par l'œil concernant le réflexe pupillaire à la lumière d'au moins un œil de l'utilisateur et/ou la sensibilité au papillotement d'au moins un œil de l'utilisateur.

3. Procédé selon les revendications 1 ou 2, le premier paramètre physiologique médié par l'œil fourni lors de l'étape de fourniture du premier paramètre physiologique médié par l'œil et/ou le deuxième paramètre physiologique médié par l'œil fourni lors de l'étape de fourniture du deuxième paramètre physiologique médié par l'œil étant déterminés par au moins un test choisi parmi la liste de tests constitué de :
- la détermination de la réponse de la pupille transitoire ;
- la détermination de la latence au rétrécissement ;
- la détermination de l'amplitude de rétrécissement ;
- la détermination de l'état de post-éclairage ;
- la détermination du diamètre de la pupille après décalage de la lumière ;
- la détermination de la réponse de la pupille en phase ;
- la détermination de la récupération de bâtonnets rétiniens lors de l'adaptation à l'obscurité ;
- la détermination de la récupération de cônes rétiniens lors de l'adaptation à l'obscurité ;
- la détermination du seuil de détection de papillotement.

4. Procédé selon l'une quelconque des revendications précédentes, comprenant en outre :
- une étape de fourniture d'un troisième paramètre physiologique médié par l'œil (S22), au cours de laquelle un troisième paramètre physiologique médié par l'œil indiquant une troisième perception ou un troisième comportement médié par l'œil de l'utilisateur est fourni,
le score de risque d'une maladie de l'œil étant déterminé en considérant le troisième paramètre physiologique médié par l'œil.

5. Procédé selon la revendication 4, le troisième paramètre physiologique médié par l'œil concernant la photosensibilité d'au moins un œil de l'utilisateur et/ou la sensibilité à la couleur d'au moins un œil de l'utilisateur et/ou la sensibilité au contraste d'au moins un œil de l'utilisateur et/ou la récupération après éblouissement d'au moins un œil de l'utilisateur et/ou la sensibilité à la déformation de forme d'au moins un œil de l'utilisateur et/ou l'acuité visuelle d'au moins un œil de l'utilisateur et/ou la sensibilité au contraste spatial d'au moins un œil de l'utilisateur et/ou la densité de pigment maculaire d'au moins un œil de l'utilisateur.

6. Procédé selon les revendications 4 ou 5, le troisième paramètre physiologique médié par l'œil fourni lors de l'étape de fourniture du troisième paramètre physiologique médié par l'œil étant déterminé par au moins un test choisi parmi la liste de tests constitué de :
- la détermination de l'acuité visuelle ;
- la détermination du seuil de photosensibilité ;
- la détermination du seuil de contraste des cônes rétiniens ;
- la détermination de la récupération de cônes rétiniens lors d'une récupération après éblouissement.

7. Procédé selon la revendication 1, le paramètre du profil de vie de l'utilisateur concernant l'âge de l'utilisateur et/ou le sexe de l'utilisateur et/ou la situation professionnelle de l'utilisateur et/ou la situation personnelle de l'utilisateur et/ou le lieu de vie de l'utilisateur et/ou l'état de santé général de l'utilisateur et/ou l'historique de santé de l'utilisateur et/ou l'historique de santé de la famille de l'utilisateur et/ou les habitudes alimentaires de l'utilisateur et/ou les habitudes d'activité physique de l'utilisateur et/ou le rythme de vie de l'utilisateur.

8. Procédé selon l'une quelconque des revendications précédentes, comprenant en outre :
- une étape de fourniture de paramètres d'environnement (S26), au cours de laquelle un paramètre d'environnement indiquant l'environnement de l'utilisateur est fourni,
le score de risque d'une maladie de l'œil étant déterminé en considérant le paramètre d'environnement.

9. Procédé selon la revendication 8, le paramètre de l'environnement de l'utilisateur concernant l'exposition à la lumière de l'utilisateur et/ou la radiance et/ou l'émission spectrale et/ou la distribution spatiale et/ou l'historique d'exposition de la lumière reçue par l'utilisateur.

10. Procédé selon l'une quelconque des revendications précédentes, comprenant en outre :
- une étape (S28) de fourniture d'un paramètre d'imagerie oculaire, au cours de laquelle un paramètre d'imagerie oculaire indiquant une imagerie oculaire de l'au moins un œil de l'utilisateur est fourni,
le score de risque d'une maladie de l'œil étant déterminé en considérant le paramètre d'imagerie oculaire.

11. Procédé selon la revendication 10, le paramètre d'imagerie oculaire concernant au moins une caractéristique de druses d'un œil de l'utilisateur, ladite caractéristique comprenant au moins l'un parmi :
la taille et la forme des druses, la surface totale des druses et l'emplacement des druses.

12. Procédé selon l'une quelconque des revendications précédentes, comprenant en outre :
- une étape de mise à jour (S40), au cours de laquelle le premier paramètre physiologique médié par l'œil et/ou le deuxième paramètre physiologique médié par l'œil et/ou le troisième paramètre physiologique médié par l'œil et/ou les données de profil de vie et/ou les données d'environnement et/ou le paramètre d'imagerie oculaire sont mis à jour,
le score de risque d'une maladie de l'œil étant déterminé en considérant les données mises à jour.

13. Système pour l'exécution du procédé de détermination d'un score de risque d'une maladie de l'œil pour un utilisateur selon l'une quelconque des revendications 1 à 12.

14. Programme adapté pour réaliser le procédé pour déterminer un score de risque d'une maladie de l'œil pour un utilisateur selon l'une quelconque des revendications 1 à 12 lorsqu'il est installé et exécuté dans le système selon la revendication 13.
